# DEMANDE DE BREVET EUROPEEN

(11) **EP 1 245 145 A1**
(43) Date de publication de la demande: **02.10.2002**
(21) Numéro de dépôt: 02003723.0
(22) Date de dépôt: 19.02.2002
(51) Int. Cl.: A01G 9/16, A01K 67/033

(54) **Dispositif pour la production de végétaux et l'élevage de petits animaux**

(30) Priorité: 29.03.2001 BE 200100210
(71) Demandeur: Hiez, Eric, 62450 Rocquigny (FR); Minet, Andréa, 62450 Rocquigny (FR); Gantier, Emmanuel, 62121 Hamelincourt (FR)
(72) Inventeur: Hiez, Eric, 62450 Rocquigny (FR)
(74) Mandataire: Hennion, Jean-Claude

(57) **Abrégé**

Dispositif pour la production de végétaux permettant également la production de petits animaux, caractérisé en ce qu'il comprend au moins un module pourvu, en combinaison :
- d'une structure porteuse formée d'au moins deux profilés formant longerons et au moins deux profilés de traverse, cette structure porteuse définissant ainsi des logements (9) dans lesquels peuvent être disposés et portés des casiers (10) à fonds perforés (11) ;
- d'un capotage de protection recouvrant ladite structure porteuse (8) ;
- des moyens permettant, lors du levage de la structure porteuse (8) de découper les radicelles des plantes contenues dans les casiers (10) à fonds perforés (11), ces radicelles ayant permis aux plantes contenues dans ces casiers (10) de puiser directement des éléments nutritifs dans un sol sur lequel les fonds perforés (11) des casiers sont posés avant le levage de la structure porteuse (8).

## Description

L'invention se rapporte à un dispositif pour la production sous abri de végétaux, en particulier des endives, ce dispositif permettant également la production de petits animaux tels que lapins, escargots, grenouilles ou coccinelles.

L'invention se rapporte plus particulièrement à des perfectionnements et aménagements aux dispositifs décrits dans les demandes de brevet français de la demanderesse, publiés sous les numéros FR-A-2 807 915, FR-A-2 807 916 et FR-A-2 807 917, dont le contenu est incorporé ici par référence.

L'endive, parfois dénommée chicorée belge, chicon ou witloof, avant d'être le légume que l'on sait, est une chicorée, plante de la famille des composées, pour laquelle on distingue trois espèces cultivées : Cichorium endivia, Cichorium intybus et Cichorium spinosum, les deux premières étant les plus utilisées en agronomie.

Cichorium endivia comprend les chicorées scaroles et frisées, sa culture remontant à l'antiquité. Chicorium intybus ou chicorée amère dite witloof est l'espèce répandue dans les régions tempérées et est utilisée depuis des siècles pour l'alimentation et comme plante médicinale.

Par endive, on désignera ici également les chicorées sauvages améliorées (type rouge Radicchio, type panachée, type à feuilles vertes et à couper telles que chicorée pain de sucre ou barbe de capucin, ainsi que les cultivars pour la consommation en frais ou après cuisson).

Dans son cycle de vie naturel, la chicorée est une plante bisannuelle qui forme, en première année, dans sa phase végétative, une racine pivotante et une rosette de feuille. Cette première phase dure environ 150 jours pour Cichorium intybus. Dans sa deuxième année, la chicorée développe dans sa phase générative une hampe florale de laquelle sont issues les graines, cette phase générative faisant suite à une dormance au printemps et de vernalisation sous l'effet du froid hivernal.

Dans le cycle de la plante du genre endive witloof, la formation de l'endive est une évolution provoquée artificiellement par rapport au cycle naturel de la plante décrit ci-dessus. Cette évolution provoquée aurait été observée pour la première fois, à la fin du 19^{ème} siècle, par M. Béziers, chef de culture au Jardin Botanique de Bruxelles. Il s'agit de la formation d'un bourgeon hypertrophié feuillu de couleur jaune-blanchâtre sur la racine de chicorée couverte de terre et privée de lumière, ce bourgeon ou chicon étant le légume vendu le plus souvent sous le nom d'endive.

Depuis que les endives ont fait leur apparition dans les marchés publics en Belgique en 1867, et à Paris vers 1879, les techniques de culture ont considérablement changé, principalement en vue de diminuer la main d'oeuvre et de produire des chicons de forme et qualités régulières.

Dans les premiers temps, les racines récoltées en automne étaient entreposées dans des caves et couvertes de terre pour faire pousser le bourgeon.

Puis, la technique des couches chaudes est apparue. Selon cette technique, le producteur doit creuser une fosse de 10cm de profondeur environ pour y loger les racines d'endives, le système de chauffage tant placé à environ 35cm de profondeur.

En 1963, des essais ont été entrepris pour produire des endives sans terre de couverture dans des chambres de forçage, cette technique s'étant développée principalement en France et aux Pays-Bas.

La culture des endives représente environ 40 000 ha dans le monde, dont 18 000 en France, la grande majorité des endives étant produites sans terre de couverture dans des salles climatisées, à partir de cultivars sélectionnés, ce forçage étant réalisé à l'abri de lumière et durant environ trois semaines.

Ce mode de culture présente de nombreux avantages :
- grâce à la conservation des racines en chambre froide, il est possible de « planter » tous les jours les endives dans les bacs de culture hydroponique pour leur forçage, et ainsi de produire des endives toute l'année durant, cette production régulière évitant le recours massif à la main d'oeuvre imposé par la technique ancestrale de culture sous couche de terre, dans laquelle toutes les endives de l'année parvenaient à maturité dans une courte période de temps ;
- grâce à la conservation des racines en chambre froide, il est possible de désinfecter la forcerie entre chaque cycle de production ;
- du fait de l'espacement imposé entre les racines, les endives obtenues présentent peu d'irrégularités, ce qui est favorable à leur classement en catégorie Extra tel que définie dans le règlement communautaire n°1872/91 du 28 juin 1991, le consommateur étant par ailleurs habitué à trouver en linéaire des endives calibrées et de forme régulière ;
- les endives sont placées dans des bacs empilés que l'on peut déplacer et transporter à l'aide d'un chariot élévateur ou équivalent ;
- les endives n'étant pas recouvertes de terre, leur nettoyage est facilité par rapport à la technique ancestrale.

Ce mode de culture présente toutefois des inconvénients.

Tout d'abord, pour maintenir une bonne température et une bonne hygrométrie dans la forcerie, des brumisateurs doivent être placés dans le système de ventilation et une centrale frigorifique doit assurer le contrôle du dégagement de chaleur des racines lors de la pousse.

Par ailleurs, les bacs de culture doivent être lavés pour contrôler les attaques bactériennes (Erwinia), ce lavage étant souvent réalisé avec des produits nocifs pour l'environnement. Les substrats de culture sont souvent très éloignés de ce qu'un consommateur peut imaginer.

Enfin, la production obtenue s'avère souvent de faible qualité en terme de saveur, le producteur ne pouvant valoriser la provenance de l'endive, la notion de terroir étant gommée par l'emploi de forceries hydroponiques et de solutions nutritives standardisées.

Le document US-A-3 879 892 décrit ainsi une technique de forçage des endives dans laquelle les racines d'endives sont placées hors sol, dans une solution nutritive, tandis que la partie supérieure de ces racines d'endives est encapuchonnée dans une poche en plastique rigide et opaque.

Le document US-A-3 935 673 décrit une technique de forçage des endives (Cichorium intybus, dite witloof). Selon cette technique antérieure, les racines d'endives sont plantées dans des boites empilées placées dans une chambre à température et humidité contrôlées. Le substrat de pousse contenu dans les boîtes comprend une mousse de polyuréthane, de la perlite ou des cendres volcaniques, une solution hydroponique étant versée sur ce substrat. Après stérilisation chimique, le substrat peut être réutilisé.

La demanderesse a constaté qu'il existe une demande croissante des consommateurs pour un retour :
- à une culture plus respectueuse de l'environnement, l'endive devant être elle aussi un produit « bio » ;
- à une endive présentant de meilleures qualités organoleptiques et un goût plus original et moins standardisé.

Les producteurs d'endives sont pour leur part certes désireux de combler les désirs de la clientèle, mais doivent tenir compte de nombreuses contingences :
- un retour au mode de culture ancestral est impensable, ne serait-ce que du fait de la main d'oeuvre qui était nécessaire ;
- les forceries représentent des investissements élevés, alors que les prix de vente de l'endive, notamment à la grande distribution, sont tirés vers le bas.

La demanderesse a ainsi identifié un besoin pour une technique de forçage qui soit tout à la fois respectueuse de l'environnement, peu coûteuse dans sa mise en place et dans son usage, peu gourmande de main d'oeuvre, cette technique permettant la production d'endives de qualité, reflétant les propriétés du terroir et assurant ainsi un argument de vente au producteur, notamment de produits « bio ».

La demanderesse a par ailleurs identifié le besoin d'une technique simplifiée d'élevage de petits animaux, tels que coccinelles, notamment pour la lutte biologique contre les parasites. Pour de tels petits animaux, l'éleveur souhaite disposer d'abris transportables, pouvant être disposés sur un sol adapté à l'animal et permettant l'élevage en toute saison, indépendamment des conditions climatiques.

La demanderesse s'est proposée de combler ces deux besoins distincts, par un nouveau dispositif ainsi que son procédé de mise en oeuvre.

A ces fins, l'invention se rapporte, selon un premier aspect, à un dispositif pour la production de végétaux permettant également la production de petits animaux, caractérisé en ce qu'il comprend au moins un module pourvu, en combinaison :
- d'une structure porteuse formée d'au moins deux profilés formant longerons et au moins deux profilés de traverse, cette structure porteuse définissant ainsi des logements dans lesquels peuvent être disposés et portés des casiers à fonds perforés ;
- d'un capotage de protection recouvrant ladite structure porteuse ;
- des moyens permettant, lors du levage de la structure porteuse, de découper les radicelles des plantes contenues dans les casiers à fonds perforés, ces radicelles ayant permis aux plantes contenues dans ces casiers de puiser directement des éléments nutritifs dans un sol sur lequel les fonds perforés des casiers sont posés avant le levage de la structure porteuse.

Ce dispositif présente, selon diverses réalisations, les caractéristiques suivantes, éventuellement combinées :
- les moyens permettant de découper les radicelles comprennent un fil, ou une lame déplacée sensiblement parallèlement et en dessous des fonds perforés des casiers, la structure support étant pourvue de moyens guide fil ou guide lame ;
- les moyens permettant de découper les radicelles comprennent un fil ou une lame guidés en translation sensiblement perpendiculairement aux longerons de la structure support ;
- les moyens permettant de découper les radicelles comprennent un fil ou une lame guidés en rotation autour d'un axe sensiblement perpendiculaire aux longerons de la structure porteuse ;
- l'axe de rotation est sensiblement disposé à mi largeur de la structure support ;
- le module comprend en outre un module technique regroupant en tout ou partie une pompe d'irrigation et une réserve d'eau d'irrigation, une pompe de circulation pour un circuit de fluide caloporteur et une réserve de fluide caloporteur, un groupe froid et un groupe chaud ;
- selon l'utilisation, au moins un module est pourvu de moyens d'aération tels que trappe à ouverture réglable ménagée sur le capotage ou ventilateur ;
- le capotage d'isolation et de protection comprend une première paroi supérieure amovible, et deux ou quatre parois latérales ;
- la première paroi supérieure amovible et les parois latérales sont sensiblement ou totalement opaques et thermiquement isolantes ;
- chacun des modules de culture ou d'élevage est pourvu d'un conduit d'alimentation en eau d'irrigation ;
- les profilés formant longerons pour la structure porteuse comprennent ou supportent des tuyaux de circulation de fluide caloporteur ;
- au moins un profilé formant traverse pour la structure porteuse comprend ou supporte un tuyau de circulation de fluide caloporteur ;
- les profilés formant longerons et/ou les profilés formant traverses sont télescopiques de sorte à permettre la mise en place de casiers de tailles variables ainsi qu'un rangement compact de la structure porteuse en période de non utilisation ;
- des vannes d'arrêt sont placées aux extrémités d'entrée et sortie des tuyaux de circulation de fluide caloporteur, permettant la désolidarisation des modules sans interrompre la circulation du fluide caloporteur ;
- la structure porteuse est solidaire du capotage de protection, notamment lors des opérations de soulèvement et de transport des modules ;
- le dispositif comprend un système d'irrigation apte à assurer par ailleurs une régulation de température dans les modules, ce système d'irrigation/régulation de température comprenant une réserve d'eau à air libre, une pompe de circulation, une électrovanne, des jets d'irrigation fonctionnant à partir d'une pression seuil déterminée, par exemple trois bars, l'électrovanne étant fermée ou ouverte suivant que le système doit être utilisé respectivement en irrigation ou régulation de température, des moyens chauffant ou refroidissant la réserve d'eau en fonction de la température désirée dans les modules.

D'autres objets et avantages de l'invention apparaîtront au cours de la description suivante de modes de réalisation, description qui va être effectuée en se référant aux dessins annexés, dans lesquels :
- la figure 1 est une vue schématique de dessus d'une installation selon un mode de réalisation ;
- la figure 2 est une vue en perspective d'un module de forçage selon un mode de réalisation ;
- la figure 3 est une vue en perspective d'un module de forçage selon un mode de réalisation, le capot de protection et d'isolation n'étant pas représenté ;
- la figure 4 est une vue en coupe transversale d'un module de forçage selon un mode de réalisation ;
- les figures 5 et 6 sont des schémas explicatifs du fonctionnement du système d'irrigation et régulation de température ;
- la figure 7 est un schéma illustrant le transport de module de forçage ;
- la figure 8 est un schéma illustrant un dispositif de découpe des radicelles, dans un mode de réalisation.

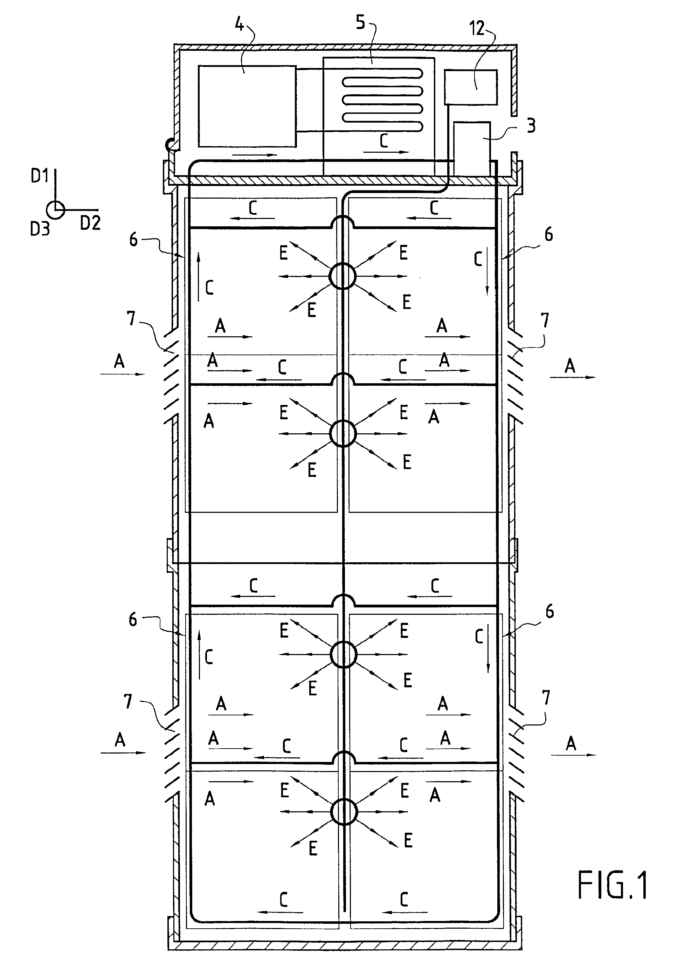

On se reporte tout d'abord à la figure 1.

L'installation vue en figure 1 comprend un module technique 1 regroupant, dans un caisson compact parallélépipédique raccordé à une source de courant électrique :
- une pompe d'irrigation et une réserve d'eau d'irrigation 2 ;
- une pompe de circulation pour le circuit de fluide caloporteur et une réserve de fluide caloporteur 3 ;
- un groupe froid 4 ;
- un groupe chaud 5 ;
- une armoire électrique de commande.

Le caisson du module technique est pourvu d'un capot articulé supérieur permettant l'accès à l'ensemble des organes listés ci-dessus, par exemple pour la maintenance ou l'entretien de ces organes.

L'installation vue en figure 1 comprend plusieurs modules de culture 6, huit modules de culture 6 étant représentés en figure 1, disposés suivant deux rangées. Ainsi que le comprendra l'homme du métier, l'installation peut comprendre un, deux ou plus de deux modules de culture, disposés suivant une, deux ou plus de deux rangées, parallèles ou non.

Un fluide caloporteur circule dans de conduits, suivant les flèches C.

L'eau d'irrigation est dispersée au-dessus des modules de culture 6, suivant les flèches E.

Une ventilation naturelle est assurée dans mes modules 6, suivant les flèches A.

A cette fin :
- les modules de culture 6 sont pourvus de trappes d'aération 7 à ouverture réglable laissant pénétrer l'air extérieur dans les modules 6 ;
- les modules de culture 6 sont conçus, ainsi qu'il apparaîtra plus complètement par la suite, de sorte à pouvoir être facilement emboîtés de manière sensiblement étanche entre eux ou totalement indépendants.

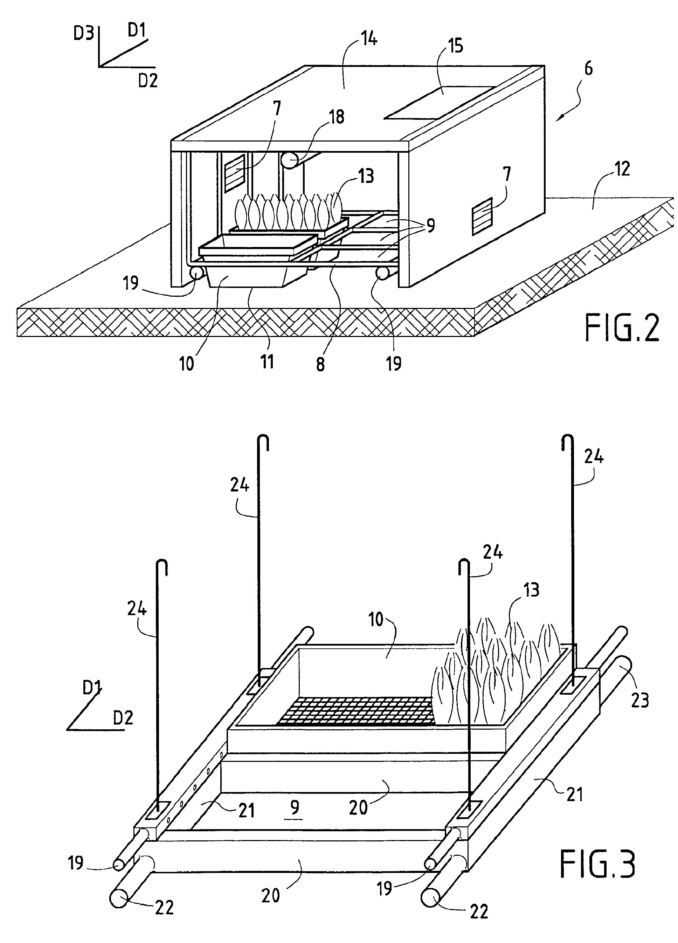

On se reporte maintenant à la figure 2.

Le module de culture 6 représenté en perspective sur la figure 2 comprend une structure porteuse ou structure support 8 en treillis de poutrelles ou profilés assemblés, cette structure porteuse 8 formant ainsi des logements 9 dans lesquels sont placés des casiers de culture 10 à fond 11 perforé. Ainsi qu'il apparaît sur la figure 2, lorsque les casiers sont en place dans les logements 9, leurs fonds perforés 11 sont en contact avec le sol 12, de sorte que les racines et radicelles des plantes contenues dans ces casiers, telles que notamment des endives 13, peuvent puiser les éléments nutritifs et l'eau directement dans le sol nourricier 12. Le dispositif permet ainsi une culture d'endives de terre. Les casiers 10 peuvent être réalisés en alliage métallique ou en matériau polymère.

Lorsque le module 6 est employé comme module d'élevage d'animaux, les casiers 10 peuvent être omis.

Le module de culture comprend en outre des parois de protection et d'isolation à savoir :
- une première paroi supérieure 14, sensiblement horizontale ou bombée, comprenant une trappe de visite 15, cette première paroi supérieure 14 étant en matériau rigide et sensiblement ou totalement opaque lorsque le module est employé pour le forçage des endives 13, ou bien sensiblement transparent lorsque le module est employé pour la culture d'autres plantes ou l'élevage d'animaux ;
- deux ou quatre parois latérales 16,17 inclinées ou sensiblement verticales et parallèles entre elles, ces parois étant pourvues de trappes d'aération 7.

Dans le mode de réalisation de la figure 2, un tuyau d'irrigation 18 est monté solidaire de la première paroi 14, sensiblement à mi-distance des parois latérales 16,17, ce tuyau d'irrigation s'étendant suivant une première direction D1 dite longitudinale. Ce tuyau d'irrigation, pourvu de buses régulièrement espacées, permet l'arrosage schématisé par les flèches E de la figure 1.

Dans d'autres modes de réalisation, non représentés, ce tuyau d'irrigation est monté solidaire de la structure porteuse 8.

Bien évidemment, plusieurs tuyaux d'irrigation peuvent être prévus, sensiblement parallèlement entre eux, lorsque le module est de grande largeur.

Ainsi qu'il apparaît en figure 2, la structure porteuse 8 comprend sur chacun de ses deux bords latéraux, un tuyau 19 de circulation de fluide caloporteur. Ces tuyaux 19 s'étendant sensiblement suivant la première direction D1 et participent à la rigidité de la structure porteuse 8. Ces tuyaux 19 permettent la circulation du fluide caloporteur suivant les flèches C de la figure 1. La régulation de la température du fluide caloporteur est assurée par exemple par un système frigorifique à inversion de cycle 4, la production de froid pouvant être inversée en production de chaleur. En variante, cette régulation de température du fluide caloporteur peut être assurée par un système frigorifique et un thermo-plongeur associé à une réserve de fluide caloporteur réchauffée ou refroidie, la circulation dans les tubes 19 étant assurée par une pompe 3.

L'ensemble de production froid et chaud est régulé par un thermostat multifonctions (étages chaud, étage froid et zone neutre) dont la sonde de température est placée dans la réserve de fluide caloporteur.

La circulation du fluide caloporteur peut être obtenue, notamment en fonction de la largeur des modules 6 mesurée suivant D2 :
- uniquement par des tuyaux longitudinaux, notamment deux tuyaux latéraux 19 ;
- également par au moins un tuyau transversal, le circuit étant ainsi en S lorsque vu de dessus.

On se reporte maintenant à la figure 3.

La structure porteuse 8 représentée en figure 3 comprend les tuyaux latéraux longitudinaux 19 formant longerons ou supportés par des profilés longerons et, de place en place, des traverses 20 formant échelons s'étendant suivant une deuxième direction D2 dite transversale. La structure porteuse 8 comprend en outre le cas échéant des profilés longitudinaux de renfort 21 par exemple en I ou en H dont les parties extrêmes opposées 22,23 sont telles que les modules 6 peuvent être assemblés par emmanchement ou emboîtement.

Ainsi que le comprendra l'homme du métier, en fonction du matériau employé pour la réalisation de la structure porteuse 8, l'assemblage des modules pourra être obtenu de différentes manières :
- par exemple assemblage à enture en croix, aboutage tourillonné lorsque la structure support 8 est réalisée en bois ou tout autre matériau analogue ;
- par exemple assemblage à fourche ou à oreilles, encliquetage lorsque la structure support est réalisée en matériau polymère ou en alliage métallique.

Des raccords rapides permettent la connexion des tuyaux 19 de circulation de fluide caloporteur, entre chaque module. Des vannes d'arrêt disposées entre chaque module sur les tuyaux 19 permettent de désolidariser un module 6 d'extrémité sans affecter l'environnement des modules restant, la circulation du fluide caloporteur n'étant pas interrompue dans les modules restant lorsque le module d'extrémité est désolidarisé. Il n'y a pas non plus de perte de fluide caloporteur lors de ce démontage du module d'extrémité.

Le producteur peut ainsi récolter le contenu des modules, un à un, en partant du module 6 le plus éloigné du module technique 1 et se rapprochant de ce module technique 1.

Des crochets 24 permettent de soulever, par exemple à l'aide d'élingues, l'ensemble formé par la structure support 8 et les casiers de culture 10. Ces crochets 24 peuvent, en variante, être remplacés par des pattes d'accrochage soudées ou fixées sur les longerons 21 et permettant de soulever et porter la structure 8 à l'aide d'un palan ou d'un crochet de chariot élévateur.

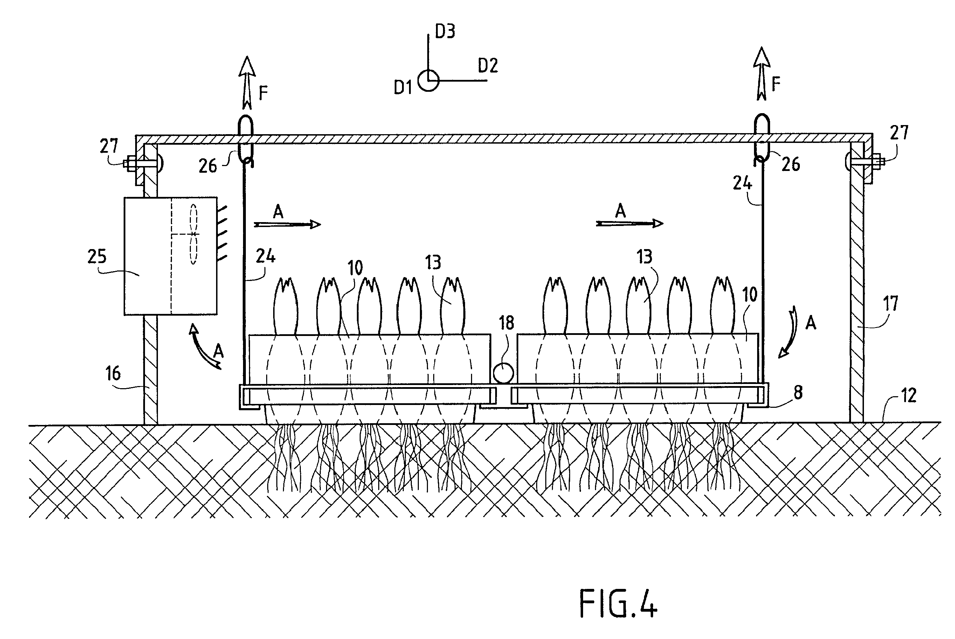

On se reporte maintenant à la figure 4.

Le mode de réalisation de la figure 4 est analogue à ceux qui viennent d'être décrits aux exceptions suivantes :
- une circulation d'air forcé est obtenue à l'aide d'un ventilateur 25 disposé sur une des parois latérales 16 du module 6 ;
- le module 6 peut être soulevé dans son ensemble selon les flèches F, la première paroi 14 et les parois latérales 16,17 étant assemblées par des moyens tels que vis ou goupilles 27 ; les crochets 24 de la structure porteuse 8 étant placés dans des oeillets 26 de pattes d'accrochage montées sur la face interne 28 de la première paroi de capotage 14 ;
- le tuyau d'irrigation 18 est monté sur la structure porteuse 8.

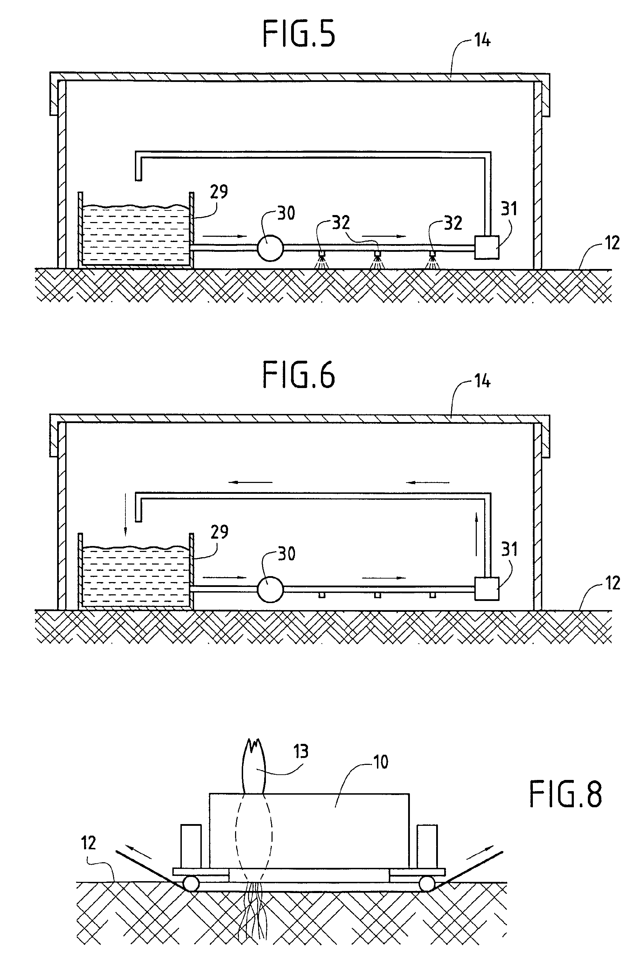

Dans un mode particulier de réalisation, le système d'irrigation est également utilisable comme système de régulation de température, selon le principe du chauffage central. Ce mode de mise en oeuvre particulier va maintenant être décrit en référence aux figures 5 et 6.

Le système d'irrigation/régulation de température comprend à cette fin :
- une réserve d'eau 29 à air libre réchauffé ou refroidi selon les besoins de la culture ou de l'élevage à réaliser dans les modules 6 ;
- une pompe de circulation 30 ;
- une électrovanne 31 positionnée en fin de circuit d'irrigation/régulation de température ;
- des jets d'irrigation 32 fonctionnant à partir d'une pression déterminée, par exemple 3 bars.

En phase d'irrigation (figure 5), l'électrovanne 31 est fermée et la pompe de circulation 30 envoie de l'eau sous pression, par exemple 4 bars, provenant de la réserve 29, l'eau s'écoulant par des jets 32, par exemple tarés à 3 bars.

En phase de régulation de température (figure 6), l'électrovanne 31 est ouverte, la pompe de circulation 30 envoie de l'eau à faible pression provenant de la réserve d'eau 29 dans le circuit d'irrigation qui fonctionne ainsi comme un circuit de régulation de température.

En fin de circuit, l'eau repart dans la réserve 29 pour y être réchauffée ou refroidie. Ce retour à l'air libre permet également l'oxygénation de l'eau, qui peut ainsi être utilisée pour l'irrigation.

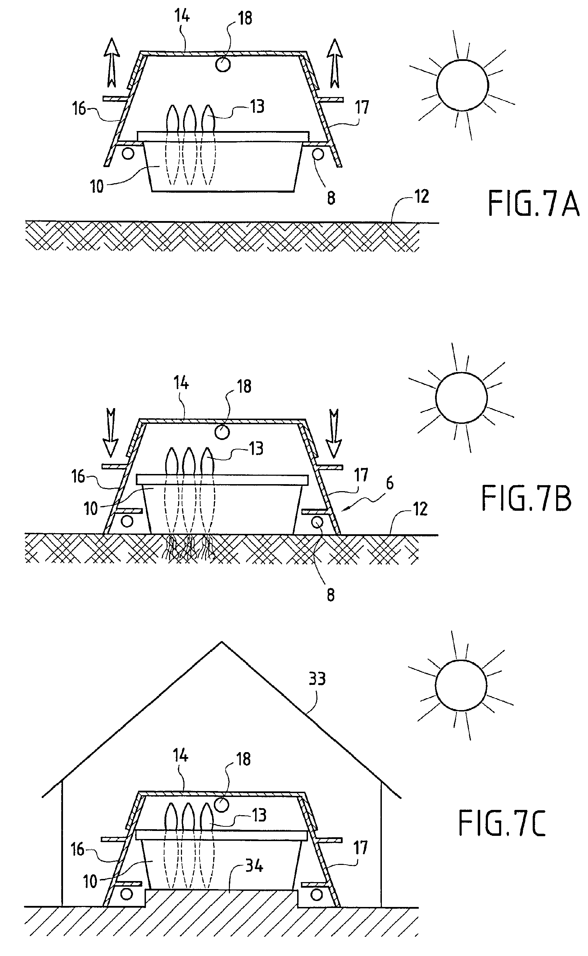

On se reporte maintenant aux figures 7 et 8.

Les modules 6, lorsqu'ils sont soulevés du sol, avec ou sans casier 10, peuvent être remisés à l'intérieur de bâtiments 33 ou dans des véhicules de transport. Ces modules 6 sont empilables avec ou sans casiers.

Lors de la récolte ou de l'entretien, les casiers 10 peuvent être soulevés et enlevés des modules 6, sans abîmer les plantes telles que les endives 13, ceci permettant d'effectuer toutes les opérations manuelles à l'abri, dans un bâtiment 33, et à bonne hauteur de travail.

Plus précisément, partant d'un module de culture 6 posé sur le sol 12, et contenant des casiers à endives 13 ou tout autre plante (figure 7b), le producteur peut transporter :
- soit la totalité du module (capot de protection 14,16,17, structure support 8 et casiers 10, (figure 7a), pour l'amener par exemple dans un bâtiment de travail 33 (figure 7c) comprenant une banquette 34 sur laquelle les casiers 10 sont déposés à bonne hauteur de travail ;
- soit uniquement tel ou tel casier 10, après avoir ouvert ou soulevé le capot de protection.

La structure support 8 peut être reliée au capotage de protection 14,16,17 par des chaînes ou élingues de sorte qu'en soulevant le capotage, le producteur soulève également la structure support 8, et par là même, les casiers 10.

Afin de pouvoir décoller du sol nourricier, soulever et transporter les modules 6, du lieu de culture situé en plein air sur terrains agricoles vers le lieu de travail situé à l'intérieur de bâtiments 33, un matériel enjambeur peut être employé. Cet enjambeur est automoteur, semi-porté ou traîné, élargi, rehaussé, équipé de pneumatiques basses pressions et d'un système d'accrochage et de levage des modules 6.

Pour obtenir une grande facilité au décollage des casiers 10 sans abîmer la culture en place et les casiers 10, la demanderesse a conçu et développé un dispositif permettant de découper les radicelles profondément implantées dans le sol 12, de sorte que le producteur n'a pas à arracher ces radicelles en soulevant les casiers, lors de la récolte ou de l'entretien.

La structure support 8 est pourvue à cette fin d'un guide fil ou d'un guide lame, tel que par exemple une gorge à ouverture vers le bas ménagée dans les longerons et/ou les traverses 20, un fil ou une lame, par exemple métallique, ayant ses extrémités logées dans cette gorge.

Dans une réalisation, le fil ou la lame est guidée en translation sensiblement suivant la direction D1, des poignées (non représentées) coulissant dans les longerons de la structure support et entraînant une lame ou un fil transversal. Deux opérateurs actionnent, le cas échéant, ce coulissement, en pressant chacun une de ces poignées.

Dans une autre réalisation, le fil ou la lame est guidée en rotation autour d'un axe sensiblement vertical et perpendiculaire aux longerons et traverses de la structure support 8, un opérateur actionnant une poignée verticale à l'extrémité de la lame ou tirant sur ledit fil.

Bien entendu, il peut être prévu de disposer un fil ou une lame sur chacun des casiers individuels 10, en plus ou au lieu d'un fil ou d'une lame commune à tous les casiers d'une structure support 8.

Les capotages de protection des modules alignés 6 peuvent être identiques et aboutés, par exemple par le biais de feuillures, rainures et languettes, tourillons, queues d'hironde, des éclisses de renfort pouvant être employées, le cas échéant.

Les capotages de protection peuvent être indépendants, les structures porteuses étant reliées par un raccord rapide.

En variante, les capotages de protection sont formés d'éléments de profilés concaves télescopiques permettant la réalisation d'un rangement très compact pendant les périodes de non utilisation.

L'invention permet la production d'endives dites de terre par opposition aux endives produites par forçage hydroponique, ces endives de terre ayant les qualités des endives obtenues par les méthodes ancestrales, sans pour autant que leur production entraîne la pénibilité et le coût de main d'oeuvre de ces méthodes ancestrales.

Les modules de culture peuvent être transportés à l'intérieur du bâtiment lors de la récolte permettant un travail à hauteur d'homme, à l'abri des intempéries.

Les modules de culture peuvent être utilisés à la fois pour l'élevage d'animaux tels que coccinelles et pour la culture de plantes. Il est ainsi possible de réduire les traitements phytosanitaires en élevant directement à proximité des plantes cultivées, des insectes luttant contre les parasites de ces plantes.

La largeur hors tout des modules 6 n'excède pas, avantageusement, la voie intérieure de l'engin de transport de ces modules (chariot élévateur par exemple), de sorte que les casiers 10 reposent sur un sol dont la structure n'est pas altérée par le passage des roues de l'engin de transport.

Pour tout nouveau cycle de culture, il suffit de porter les modules vers un sol préparé à cette fin.

La demanderesse a constaté que douze cycles de forçage d'endives par an étaient réalisables à l'aide du dispositif selon l'invention.

La demanderesse a constaté que le phénomène d'évaporation était limité par l'emploi du dispositif selon l'invention, de sorte que l'humidité relative HR est suffisamment assurée par l'humidité du sol 12 nourricier pour que les dispositifs de brumisation des forceries ne soient pas nécessaires.

A titre indicatif, un module 6 peut avoir les dimensions suivantes :
- longueur : environ quatre mètres ;
- largeur : environ deux mètres cinquante ;
- hauteur : environ un mètre.

## Revendications

1. Dispositif pour la production de végétaux permettant également la production de petits animaux, **caractérisé en ce qu'**il comprend au moins un module pourvu, en combinaison :
- d'une structure porteuse formée d'au moins deux profilés formant longerons et au moins deux profilés de traverse, cette structure porteuse définissant ainsi des logements (9) dans lesquels peuvent être disposés et portés des casiers (10) à fonds perforés (11) ;
- d'un capotage de protection recouvrant ladite structure porteuse (8) ;
- des moyens permettant, lors du levage de la structure porteuse (8) de découper les radicelles des plantes contenues dans les casiers (10) à fonds perforés (11), ces radicelles ayant permis aux plantes contenues dans ces casiers (10) de puiser directement des éléments nutritifs dans un sol sur lequel les fonds perforés (11) des casiers sont posés avant le levage de la structure porteuse (8).

2. Dispositif selon la revendication 1 **caractérisé en ce que** les moyens permettant de découper les radicelles comprennent un fil, ou une lame déplacée sensiblement parallèlement et en dessous des fonds perforés (11) des casiers (10), la structure support (8) étant pourvue de moyens guide fil ou guide lame.

3. Dispositif selon la revendication 2, **caractérisé en ce que** les moyens permettant de découper les radicelles comprennent un fil ou une lame guidés en translation sensiblement perpendiculairement aux longerons de la structure support (8).

4. Dispositif selon la revendication 2, **caractérisé en ce que** les moyens permettant de découper les radicelles comprennent un fil ou une lame guidés en rotation autour d'un axe sensiblement perpendiculaire aux longerons de la structure porteuse (8).

5. Dispositif selon la revendication 4 **caractérisé en ce que** l'axe de rotation est sensiblement disposé à mi largeur de la structure support (8).

6. Dispositif selon l'une quelconque des revendications 1 à 5 **caractérisé en ce qu'**il comprend en outre un module technique (1) regroupant en tout ou partie une pompe d'irrigation et une réserve d'eau d'irrigation (2), une pompe de circulation pour un circuit de fluide caloporteur et une réserve de fluide caloporteur (3), un groupe froid (4) et un groupe chaud (5).

7. Dispositif selon l'une quelconque des revendications 1 à 6 **caractérisé en ce que** au moins un module (6) est pourvu de moyens d'aération tels que trappe à ouverture réglable ménagée sur le capotage ou ventilateur.

8. Dispositif selon l'une quelconque des revendications 1 à 7, **caractérisé en ce que** le capotage d'isolation et de protection comprend une première paroi supérieure (14) amovible, et deux ou quatre parois latérales (16,17).

9. Dispositif selon la revendication 8 **caractérisé en ce que** la première paroi supérieure amovible (14) et les parois latérales (16,17) sont sensiblement ou totalement opaques et thermiquement isolantes.

10. Dispositif selon l'une quelconque des revendications 1 à 9 **caractérisé en ce que** chacun de ses modules de culture ou d'élevage (6) est pourvu d'un conduit d'alimentation en eau d'irrigation.

11. Dispositif selon l'une quelconque des revendications 1 à 10 **caractérisé en ce que** les profilés formant longerons pour la structure porteuse (8) comprennent ou supportent des tuyaux de circulation (18) de fluide caloporteur.

12. Dispositif selon l'une quelconque des revendications 1 à 11 **caractérisé en ce que** au moins un profilé formant traverse pour la structure porteuse (8) comprend ou supporte un tuyau de circulation (18) de fluide caloporteur.

13. Dispositif selon l'une quelconque des revendications 1 à 12 **caractérisé en ce que** les profilés formant longerons et/ou les profilés formant traverses sont télescopiques de sorte à permettre la mise en place de casiers (10) de tailles variables ainsi qu'un rangement compact de la structure porteuse (8) en période de non utilisation.

14. Dispositif selon l'une quelconque des revendications 11 à 13 **caractérisé en ce que** des vannes d'arrêt sont placées aux extrémités d'entrée et sortie des tuyaux de circulation de fluide caloporteur, permettant la désolidarisation des modules sans interrompre la circulation du fluide caloporteur.

15. Dispositif selon l'une quelconque des revendications 1 à 14 **caractérisé en ce que** la structure porteuse (8) est solidaire du capotage de protection lors des opérations de soulèvement et de transport.

16. Dispositif selon l'une quelconque des revendications 1 à 15 **caractérisé en ce qu'**il comprend un système d'irrigation apte à assurer par ailleurs une régulation de température dans les modules (6), ce système d'irrigation/régulation de température comprenant :
- une réserve d'eau (29) à air libre ;
- une pompe de circulation (30) ;
- une électrovanne (31) ;
- des jets d'irrigation fonctionnant à partir d'une pression seuil déterminée, par exemple trois bars,
l'électrovanne (31) étant fermée ou ouverte suivant que le système doit être utilisé respectivement en irrigation ou régulation de température, des moyens chauffant ou refroidissant la réserve d'eau en fonction de la température désirée dans les modules.
